(19) Europäisches Patentamt
European Patent Office
Office européen des brevets

(11) EP 4 194 433 A1

(12) **EUROPÄISCHE PATENTANMELDUNG**

(43) Veröffentlichungstag:
**14.06.2023 Patentblatt 2023/24**

(21) Anmeldenummer: **21020619.9**

(22) Anmeldetag: **07.12.2021**

(51) Internationale Patentklassifikation (IPC):
***C07C 41/01*** (2006.01) ***C07C 43/03*** (2006.01)
***B01J 8/04*** (2006.01)

(52) Gemeinsame Patentklassifikation (CPC):
(C-Sets verfügbar)
**C07C 41/09; B01J 8/04; B01J 12/00; C07C 29/153; C07C 41/01** (Forts.)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder:
- **Linde GmbH**
  **82049 Pullach (DE)**
- **BASF SE**
  **67056 Ludwigshafen am Rhein (DE)**

(72) Erfinder:
- **WINKLER, Florian**
  **82049 Pullach (DE)**
- **LANVER, Virginie**
  **82049 Pullach (DE)**
- **BOTTKE, Nils**
  **82049 Pullach (DE)**
- **GENTZEN, Manuel**
  **82049 Pullach (DE)**
- **TAURO, Silvia**
  **67063 Ludwigshafen (DE)**

(74) Vertreter: **Fischer, Werner**
**Linde GmbH**
**Intellectual Property EMEA**
**Dr.-Carl-von-Linde-Straße 6-14**
**82049 Pullach (DE)**

(54) **VERFAHREN UND ANLAGE ZUR HERSTELLUNG VON DIMETHYLETHER**

(57) Ein Verfahren zur Synthese von Dimethylether wird vorgeschlagen, bei dem ein Kohlendioxid und/oder Kohlenmonoxid sowie Wasserstoff enthaltendes Einsatzgasgemisch (E) durch mit einem ersten Katalysator und einem zweiten Katalysator ausgestattete Reaktionsrohre (1) eines gekühlten Rohrbündelreaktors (10) geführt wird, bei dem das in dem Einsatzgasgemisch (E) enthaltene Kohlendioxid und/oder das in dem Einsatzgasgemisch enthaltene Kohlenmonoxid zumindest zum Teil an dem ersten Katalysator mit dem in dem Einsatzgasgemisch enthaltenen Wasserstoff zu Methanol umgesetzt wird, und bei dem in demselben Rohrbündelreaktor (10) das Methanol zumindest zum Teil an dem zweiten Katalysator zu dem Dimethylether umgesetzt wird. Es ist vorgesehen, dass die Reaktionsrohre (1) mit dem ersten und dem zweiten Katalysator in Form einer strukturierten Schüttung ausgestattet sind, wobei die strukturierte Schüttung zwei bis vier Bereiche (11, 12, 13) aufweist, in denen der erste und zweite Katalysator in unterschiedlichen physikalischen Mischungen bereitgestellt sind, wobei die Bereiche (11, 12, 13) derart angeordnet sind, dass die Konzentration des ersten Katalysators in einer Strömungsrichtung durch die Reaktionsrohre (1) zunimmt. Ferner wird eine Anlage (100) zur Durchführung eines solchen Verfahrens vorgeschlagen.

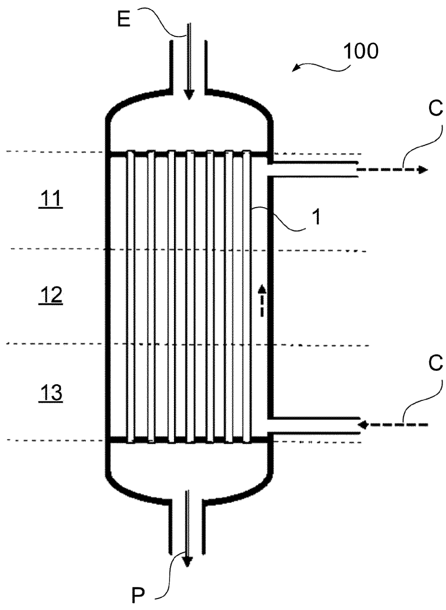


**Fig. 1**

EP 4 194 433 A1

(52) Gemeinsame Patentklassifikation (CPC): (Forts.)

C-Sets
**C07C 29/153, C07C 31/04;**
**C07C 41/01, C07C 43/043;**
**C07C 41/09, C07C 43/043**

## Beschreibung

Die Erfindung betrifft ein Verfahren und eine Anlage zur Herstellung von Dimethylether aus Synthesegas gemäß den Oberbegriffen der unabhängigen Patentansprüche.

### Hintergrund der Erfindung

Bei Dimethylether (DME) handelt es sich um den strukturell einfachsten Ether. Er enthält zwei Methylgruppen als organische Reste, ist polar und findet für unterschiedliche Zwecke in der Industrie Verwendung.

Dimethylether kann mittels einer zweistufigen Synthese aus Synthesegas über das Intermediat Methanol hergestellt werden, wie beispielsweise in Kapitel 4 des DME Handbook, Japan DME Forum, Tokyo 2007, ISBN 978-4-9903839-0-9, beschrieben. Eine "zweistufige" Synthese zeichnet sich dadurch aus, dass zunächst Methanol aus Synthesegas hergestellt wird, wobei das Methanol von nicht umgesetztem Synthesegas abgetrennt und das Methanol anschließend separat und in einem weiteren Schritt zu Dimethylether und Wasser dehydratisiert wird.

Bei einer einstufigen Synthese von Dimethylether laufen dagegen sämtliche Reaktionen in einunddemselben Reaktor und in einunddemselben Katalysatorbett ab. Nachfolgend wird für eine einstufige Synthese von Dimethylether auch der Begriff Direktsynthese verwendet. Bei den ablaufenden Reaktionen handelt es sich um eine Reaktion von Wasserstoff mit Kohlenstoffmonoxid und/oder Kohlenstoffdioxid zu Methanol und eine (Weiter-)Reaktion von Methanol zu Dimethylether und Wasser. Die einstufige Synthese von Dimethylether ist beispielsweise aus der US 4,536,485 A und der US 5,189,203 A bekannt. Herkömmlicherweise werden hierbei Hybridkatalysatoren verwendet. Die Reaktion ist exotherm und erfolgt typischerweise bei einer Temperatur von 200 bis 300°C und bei einem Druck von 20 bis 100 bar.

Insbesondere in Bezug auf die nachfolgend verwendete Terminologie, aber auch auf grundsätzliche Überlegungen die Synthese von Dimethylether aus Synthesegas betreffend, sei auf den Artikel von I. Kiendl et al., Chem. Ing. Tech. 2020, 92, No. 6, 736-745, verwiesen. Die Begriffe werden hier entsprechend verwendet.

Das für die Herstellung von Dimethylether erforderliche Synthesegas kann durch eine Vielzahl geeigneter Technologien, wie sie beispielsweise bei Hiller et al. im Artikel "Gas Production" in Ullmann's Encyclopedia of Industrial Chemistry, Onlineausgabe 15. Dezember 2006, doi 10.1002/14356007.a12_169.pub2, beschrieben sind, und aus einer Vielzahl von Ausgangsmaterialien bereitgestellt werden.

Bei der zweistufigen Synthese von Dimethylether können für die Umsetzung zu Methanol kupferhaltige Katalysatoren (nachfolgend auch als "Methanolkatalysatoren" bezeichnet) und für den Folgeschritt der Dehydratisierung insbesondere saure Katalysatoren wie Zeolithkatalysatoren, Katalysatoren auf Grundlage von $\gamma$-Dialuminiumtrioxid und Alumosilikatalysatoren (nachfolgend auch als "Dehydratisierungskatalysatoren" bezeichnet) eingesetzt werden. Das Synthesegas zur Erzeugung von Methanol zeichnet sich dabei durch eine Stöchiometriezahl (zur Definition wird ausdrücklich auf Kiendl et al. verwiesen) von etwas mehr als 2 aus.

Die zweistufige Dimethylethersynthese stellt Dimethylether mit hoher Reinheit her. Das Methanol aus der ersten Stufe wird von höheren Alkoholen befreit und dann hoch selektiv dehydratisiert. In einem einstufigen Verfahren sind Nebenprodukte eine weitere Herausforderung, da hier die Methanolsynthese und Dimethylethersynthese parallel verlaufen und die entstehenden höheren Alkohole zu Olefinen umgesetzt werden und je nach Siedelage im Produkt auftreten.

Die einstufige Dimethylethersynthese ist industriell noch nicht realisiert. Dies liegt vor allem an der Schwierigkeit, die beiden Katalysatoren in einem Reaktor zu integrieren. Im gemeinsamen Reaktor liegen andere Temperatur- und Partialdruckbedingungen der Edukte und Produkte als in den Einzelreaktoren vor, wodurch eine Temperaturführung der Reaktion wesentlich erschwert ist und die Alterung der Katalysatoren beispielsweise durch erhöhte Konzentrationen bzw. Partialdrücke der Nebenprodukte beschleunigt ist. Möglichkeiten einer Temperaturkontrolle in der Direktsynthese von Dimethylether sind in der WO 2019/122078 A1 beschrieben.

Aufgrund der höheren Temperaturen und erhöhten Wasserpartialdrücke im Vergleich zur konventionellen Methanolsynthese neigen Kupferkatalysatoren unter diesen eher zum Sintern. Weiterhin wird in der Literatur durch das Ablaufen von Hydroxylierungsreaktionen der Nebenphasen im Kupferkatalysator eine erhöhte Mobilität zugesprochen (A. Prašnikar et al., Ind. Eng. Chem. Res., 2019, 58, 13021) Dadurch kann auch die Mobilität der Kupferpartikel weiter erhöht werden. Weiterhin kann dadurch ein Bedecken der Kupferpartikel erfolgen, wodurch die aktive Oberfläche ebenfalls reduziert wird. Die Kontrolle der Parameter Temperatur und Konzentration/Partialdrücke stellt daher eine entscheidende Herausforderung in der Realisierung der direkten Dimethylethersynthese dar.

In der Dimethylethersynthese setzt sich Wasserstoff und Kohlenmonoxid mittels folgender Reaktionen um:

$$2\ H_2 + CO \rightleftharpoons MeOH$$

$$2\ MeOH \rightleftharpoons DME + H_2O$$

$$CO + H_2O \rightleftharpoons CO_2 + H_2$$

Es handelt sich hierbei um Gleichgewichtsreaktionen, die je nach Druck, Temperatur und Feedzusammensetzung eine andere Produktzusammensetzung ergeben.

Bei einem Verhältnis von Wasserstoff zu Kohlenmonoxid von 1 ohne Kohlendioxid im Feedgas beobachtet man die folgende Nettoreaktion:

$$3\ H_2 + 3\ CO \rightarrow DME + CO_2$$

Bei einer Stöchiometriezahl von 2 dominiert folgende Nettoreaktion:

$$2\ H_2 + CO \rightarrow DME + H_2O$$

Höhere Alkohole können über eine Kettenverlängerung über eine Insertion von Kohlenmonoxid gebildet werden. Diese Reaktion findet am Kupferkatalysator (Kat) statt. Die Insertion erfolgt an Methylspezies, die durch Hydrierung von adsorbiertem Kohlenmonoxid bzw. durch Readsorption von Methanol und einer vorherigen Spaltung der Kohlenstoff-Sauerstoff-Bindung entstehen. Eine weitere Hydrierung der adsorbierten C2-Spezies führt dann zur Bildung von Acetaldehyd bzw. nach schneller Hydrierung zur Bildung von Ethanol.

$$2\ CH_3OH + Kat\text{-}OH \rightarrow CH_3O\text{-}Kat + H_2O$$

$$CH_3O\text{-}Kat + CO \rightarrow CH_3COO\text{-}Kat$$

$$CH_3COO\text{-}Kat + H_2 \rightarrow C_2H_5OH$$

Die höheren Alkohole werden zu den korrespondierenden Olefinen dehydratisiert und gegebenenfalls zu Paraffinen hydriert.

Einige Studien unterstützen auch durch DFT-Rechnungen die Bildung der Kohlenstoff-Kohlenstoff-Bindung durch eine Insertion von Kohlenmonoxid in adsorbierte Methylspezies, die beispielsweise aus Hydrierung von adsorbiertem Kohlenmonoxid entstehen. Auch die Adsorption von Methanol kann nach Spaltung der Kohlenstoff-Sauerstoff-Bindung wieder über eine Insertion von Kohlenmonoxid zur Bildung von Ethanol führen. Ein höherer Partialdruck von Methanol könnte daher auch zu einer vermehrten Bildung von Nebenprodukten führen (siehe z.B. Z.-J. Zuo et al., J. Phys. Chem. C 2014, 118, 12890). Weitere Studien an modifizierten Kupferkatalysatoren belegen darüber hinaus auch die Bildung von Ethanol durch eine Kopplung zweier Methanolmoleküle (J.J. Spifey & A. Egbebi, Chem. Soc. Rev.,2007, 36, 1514).

Eine weitere Nebenreaktion, die bei Zeolithen auftritt, ist die Bildung von Olefinen aus Methanol und/oder Dimethylether. Diese kann bereits ab Temperaturen deutlich unter 300°C, stattfinden und richtet sich neben der Temperatur auch nach der Verweilzeit und dem verwendeten Katalysator. Jene Nebenreaktion folgen den aus der Literatur bekannten Mechanismen von Methanol-to-Olefins (MTO) bzw. Methanol-to-Propylene (MTP).

Bei der Direktsynthese von Dimethylether müssen im Reaktor insbesondere die folgenden Probleme gelöst werden:

- Vergleichsweise kurze Laufzeiten des Katalysators wegen erhöhter Alterung durch vorherrschenden Reaktionsbedingungen.
- Nebenprodukte im Dimethyletherprodukt wegen paralleler Methanol- und Dimethylethersynthese und somit fehlender Zwischenaufreinigung.
- Vergleichsweise niedrige Produktivität im hinteren Bett durch kinetisch kontrollierte Bedingungen und niedrige Temperaturen.
- Temperaturkontrolle der Reaktion für kontrollierte Alterung.
- Hohe Kosten durch Katalysatorwechsel und/oder Overdesign des Reaktors.

Ein Vorteil der direkten Synthese von Dimethylether ergibt sich u.a. durch die Verschiebung des Gleichgewichts. Durch eine separate Methanolsynthese im vorderen Teil des Reaktors gemäß WO 2019/122078 A1 wird dieser Vorteil

nur im hinteren Teil des Reaktors ausgenutzt. Daher sind geringere Ausbeuten in diesem Fall zu erwarten. Dies entspricht einer niedrigeren Produktivität im vorderen Teil des Reaktors.

Die vorliegende Erfindung stellt sich die Aufgabe, die erwähnten Nachteile bei bekannten Verfahren zur Direktsynthese von Dimethylether zu überwinden.

Offenbarung der Erfindung

Diese Aufgabe wird durch ein Verfahren und eine Anlage zur Herstellung von Dimethylether aus Synthesegas gemäß den jeweiligen unabhängigen Patentansprüchen gelöst. Vorteilhafte Ausgestaltungen und Weiterbildungen sind Gegenstand der Unteransprüche sowie der nachfolgenden Beschreibung.

Die vorliegende Erfindung schlägt ein Verfahren zur Synthese von Dimethylether vor, bei dem ein Kohlendioxid und/oder Kohlenmonoxid sowie Wasserstoff enthaltendes Einsatzgasgemisch durch mit einem ersten und einem zweiten Katalysator (insbesondere in Form des zuvor erwähnten Methanolkatalysators und des ebenfalls erwähnten Dehydratisierungskatalysators) ausgestattete Reaktionsrohre eines gekühlten Rohrbündelreaktors geführt wird, bei dem das in dem Einsatzgasgemisch enthaltene Kohlendioxid und/oder das in dem Einsatzgasgemisch enthaltene Kohlenmonoxid zumindest zum Teil an dem ersten Katalysator mit dem in dem Einsatzgasgemisch enthaltenen Wasserstoff zu Methanol umgesetzt wird, und bei dem in demselben Rohrbündelreaktor das Methanol zumindest zum Teil an dem zweiten Katalysator zu dem Dimethylether umgesetzt wird.

Im Rahmen der vorliegenden Erfindung können fachüblich ausgebildete Rohrbündelreaktoren eingesetzt werden. Zu weiteren Details bezüglich entsprechender Reaktoren sei auf einschlägige Fachliteratur wie beispielsweise den Artikel von G. Eigenberger, "Fixed-Bed Reactors", in Ullmann's Encyclopedia of Industrial Chemistry, Band B 4, 1992, Seiten 199 bis 238, verwiesen. Wie dort beispielsweise im Zusammenhang mit Figur 4.1 D und E beschrieben, ist zur Temperaturführung in Rohrbündelreaktoren auch die Verwendung mehrerer Kühl- und Reaktionszonen an sich bekannt. Die vorliegende Erfindung schlägt eine besonders vorteilhafte Zonierung vor, wie sie nachfolgend erläutert wird.

Erfindungsgemäß ist vorgesehen, dass die Reaktionsrohre mit dem ersten und dem zweiten Katalysator in Form einer strukturierten Schüttung ausgestattet sind, wobei die strukturierte Schüttung zwei bis vier Bereiche (synonym hier auch als Zonen bezeichnet) aufweist, in denen der erste und zweite Katalysator in physikalischen Mischungen mit unterschiedlichen Mischungsverhältnissen bereitgestellt sind, wobei die Bereiche derart angeordnet sind, dass die Konzentration des ersten Katalysators (d.h. des Methanolkatalysators) in einer Strömungsrichtung durch die Reaktionsrohre zunimmt.

Der Begriff strukturierte Schüttung (oder strukturiertes Bett) versteht sich im Rahmen der vorliegenden Erfindung insbesondere als eine Aufeinanderfolge von katalytischen Bereichen bzw. Zonen mit unterschiedlichen Anteilen bzw. Gehalten an erstem und zweitem Katalysator (Methanolkatalysator und Dehydratisierungskatalysator) in einem Reaktor. Innerhalb eines Bereichs bzw. einer Zone sind diese Anteile gleich bzw. weisen nur aufgrund herstellungstechnischer Varianzen voneinander ab. In Richtung stromabwärts ist der Anteil bzw. Gehalt des ersten Katalysators (Methanolkatalysators) von Bereich zu Bereich bzw. Zone zu Zone aufsteigend. Primär handelt es sich innerhalb eines Bereichs bzw. einer Zone des strukturierten Betts um eine physikalische Mischung aus dem ersten und dem zweiten Katalysator (Methanolkatalysator und Dehydratisierungskatalysator). Sonderform ist ein sogenannter bifunktionaler Katalysator. Hier sind beide Katalysatoren auf einem gemeinsamen Träger aufgebracht und nicht physikalisch gemischt. Die Katalysatoren können auf Füllkörpern wie Pellets, Ringen oder irregulären Körpern aufgebracht sein.

Die vorliegende Erfindung unterscheidet sich insbesondere von aus dem Stand der Technik bekannten sogenannten Vorbetten (engl. Pre Beds), in denen ein Methanolkatalysator mit Inertmaterial verdünnt ist, und dem ein Mischbett in Form einer physikalischen Mischung von Methanol- und Dehydratisierungskatalysator nachfolgt. Abweichend von einem einheitlichen Bett bzw. Einzelbett, d.h. einer einheitlichen physikalischen Mischung aus Methanol- und Dehydratisierungskatalysator den gesamten Reaktor (bzw., falls ein Vorbett verwendet wird, über den dem Vorbett nachfolgenden Bereich) ermöglicht ein strukturiertes Bett durch Reaktionsführung durch unterschiedliche Verhältnisse von Methanol- und Dehydratisierungskatalysator.

Der erste Katalysator kann im Rahmen der vorliegenden Erfindung insbesondere ein kupferbasierter Katalysator der eingangs erläuterten Art sein, so dass auf die zitierte Fachliteratur verwiesen werden kann. Der zweite Katalysator kann ebenfalls in fachüblicher Weise ausgebildet sein. Es kann sich also insbesondere um einen sauren Katalysator auf Zeolithen, $\gamma$-Dialuminiumtrioxid und Alumosilikat handeln.

Im Rahmen der vorliegenden Erfindung kann das Verfahren insbesondere umfassen, dass der Reaktor in einem Druckbereich von 30 bis 80 bar und/oder in einem Temperaturbereich von 200 bis 290°C und/oder mit einer stündlichen Gas-Raumgeschwindigkeit (GHSV) von 1.500 bis 4.000 $h^{-1}$ betrieben wird.

In einer ersten Ausgestaltung der Erfindung kann die Anzahl der Bereiche, die die Schüttung aufweist, insbesondere zwei sein (in den nachfolgenden Tabellen 1 und 2 in den Tabellenspalten 2 dargestellt), wobei ein erster der Bereiche ein stromaufwärtiger Bereich (in den Tabellen 1 und 2 mit MIX1 angegeben) von 0 bis 30% der Länge der Reaktionsrohre ist und ein zweiter der Bereiche ein stromabwärts angeordneter Bereich (MIX2) von 25 bis 100% der

Länge der Reaktionsrohre ist. (Leere Tabellenzellen entsprechen Bereichen ohne Schüttungen.)

Die hier genannten Prozentbereiche in der Tabelle entsprechen Längenbereichen des jeweiligen Reaktionsrohrs. Mit anderen Worten erstreckt sich ein mit A bis B% bezeichneter Abschnitt eines Reaktionsrohrs, das eine Länge von X cm aufweist, von einer Startposition bei A×X/100 cm bis zu einer Endposition bei BxX/100 cm. Mit nochmals anderen Worten stellen der erste und der zweite Wert der nachfolgenden Bereiche eine erste, durch die Prozentangabe ausgedrückte Längenposition des Reaktionsrohrs und eine zweite, durch die Prozentangabe ausgedrückte Längenposition des Reaktionsrohrs dar. Überlappende Bereichsangaben sind derart zu verstehen, dass ein Überlappungsbereich von entweder dem einen oder dem anderen Abschnitt gebildet sein kann.

In der ersten Ausgestaltung der Erfindung kann in dem ersten der Bereiche ein Anteil des ersten Katalysators 1 und ein Anteil des zweiten Katalysators X betragen und in dem zweiten der Bereiche kann ein Anteil des ersten Katalysators 1,5 bis 4 und ein Anteil des zweiten Katalysators X betragen.

In einer zweiten Ausgestaltung der Erfindung kann die Anzahl der Bereiche, die die Schüttung aufweist, insbesondere drei sein (Tabellenspalten 3), wobei ein erster der Bereiche (MIX1) ein stromaufwärtiger Bereich von 0 bis 30% der Länge der Reaktionsrohre ist, ein zweiter der Bereiche (MIX2) ein zentraler Bereich von 20 bis 70% der Länge der Reaktionsrohre ist, und ein dritter der Bereiche (MIX3) ein stromabwärtiger Bereich von 50 bis 100% der Länge der Reaktionsrohre ist.

In der zweiten Ausgestaltung der Erfindung kann in dem ersten der Bereiche ein Anteil des ersten Katalysators 1 und ein Anteil des zweiten Katalysators X betragen, in dem zweiten der Bereiche kann ein Anteil des ersten Katalysators 1,5 bis 2 und ein Anteil des zweiten Katalysators X betragen, und in dem dritten der Bereiche kann ein Anteil des ersten Katalysators 3 bis 4 und ein Anteil des zweiten Katalysators X betragen.

In einer dritten Ausgestaltung der Erfindung kann die Anzahl der Bereiche, die die Schüttung aufweist, insbesondere vier sein (Tabellenspalten 4), wobei ein erster der Bereiche (MIX1) ein stromaufwärtiger Bereich von 0 bis 25% der Länge der Reaktionsrohre ist, ein zweiter der Bereiche (MIX2) ein stromaufwärts angeordneter zentraler Bereich von 20 bis 50% der Länge der Reaktionsrohre ist, ein dritter der Bereiche (MIX3) ein stromabwärts angeordneter zentraler Bereich von 50 bis 75% der Länge der Reaktionsrohre ist, und ein vierter der Bereiche (MIX4) ein stromabwärtiger Bereich von 75 bis 100% der Länge der Reaktionsrohre ist.

In der dritten Ausgestaltung der Erfindung kann in dem ersten der Bereiche ein Anteil des ersten Katalysators 1 und ein Anteil des zweiten Katalysators X betragen, in dem zweiten der Bereiche kann ein Anteil des ersten Katalysators 1,5 bis 2 und ein Anteil des zweiten Katalysators X betragen, in dem dritten der Bereiche kann ein Anteil des ersten Katalysators 2,5 bis 3 und ein Anteil des zweiten Katalysators X betragen, und in dem vierten der Bereiche kann ein Anteil des ersten Katalysators 4 und ein Anteil des zweiten Katalysators X betragen.

In allen Ausgestaltungen kann X insbesondere gleich sein und 0,3 bis 1 betragen.

Die zuvor erläuterten Ausgestaltungen werden nachfolgend unter Bezugnahme auf die bereits erwähnten Tabellen nochmals veranschaulicht.

**Tabelle 1**

| Anzahl Bereiche | 2 | 3 | 4 |
|---|---|---|---|
| | Rohrlänge % | Rohrlänge % | Rohrlänge % |
| MIX1 | 0-30 | 0-30 | 0-25 |
| MIX2 | 25-100 | 20-70 | 20-50 |
| MIX3 | | 50-100 | 50-75 |
| MIX4 | | | 75-100 |

**Tabelle 2**

| Anzahl Bereiche | 2 | 3 | 4 |
|---|---|---|---|
| MIX1 | 1:X | 1:X | 1:X |
| MIX2 | 1,5-4:X | 1,5-2:X | 1,5-2:X |
| MIX3 | | 3-4:X | 2,5-3:X |
| MIX4 | | | 4:X |

Der Vorteil der direkten Synthese von Dimethylether ergibt sich u.a. durch die Erhöhung des Gleichgewichtsumsatzes. Durch separate Methanolsynthese im vorderen Teil des Reaktors gemäß dem Stand der Technik wird dieser Vorteil nur im hinteren Teil des Reaktors ausgenutzt. Diese Kombination dient der Temperaturkontrolle. Es liegt eine niedrigere Produktivität im vorderen Teil des Reaktors vor. Es hat sich nun gezeigt, dass die Vorteile einer derartigen Temperaturkontrolle durch eine geeignete Mischung der zwei unterschiedlichen Katalysatoren ebenfalls generiert werden kann. Dies hat neben den Vorteil der Temperaturkontrolle den zusätzlichen Effekt der Raumzeitausbeutensteigerung. Dies ist insbesondere durch die unten erläuterten Beispiele 1 bis 3 belegt.

Im vorderen bzw. stromaufwärtigen Teil wird durch den geringeren Anteil an dem ersten Katalysator der Mischung weniger Methanol gebildet und dadurch die Temperaturkontrolle durch die geringere Exothermie ermöglicht. Zugleich sorgt der hier vorhandene hohe Dehydratisierungskatalysatoranteil für eine verstärktere Weiterreaktion des Methanols zu Dimethylether und verhindert den Aufbau hoher Partialdrücke von Methanol, die gerade im vorderen Teil des Reaktors zusammen mit den hohen Partialdrücken von Kohlenmonoxid zur Nebenproduktbildung führen. Selbst wenn ein Gleichgewicht der Methanolreaktion erreicht werden würde, wäre hier der Anteil an dem ersten Katalysator gegenüber anderer Ausführungsformen niedriger und somit die Nebenreaktionsbildung in gleichem Maße.

Mindestens ein und maximal drei weitere anschließende Betten sind mit jeweils höheren Anteilen des ersten Katalysators auszuführen. Dies ermöglicht eine gezielte Temperaturführung der Reaktion. Dadurch können Spitzentemperaturen von über 280°C vermieden werden. Idealerweise hält man die Temperatur in einem Bereich zwischen 200°C und 280°C und besonders zwischen 220°C und 270°C. Dies verhindert ein vorzeitiges Altern durch Sintereffekte beispielsweise an der Kupferkomponente des ersten Katalysators. Die bessere Kontrolle von Temperaturspitzen ermöglicht zudem eine Vermeidung einer durch den zweiten Katalysator als Nebenreaktion katalysierte Olefinbildung bzw. von Folgereaktionen. Der höhere Anteil des ersten Katalysators im hinteren Teil des Reaktors ist durch den geringeren Partialdruck von Kohlenmonoxid hier unkritischer für die Nebenproduktbildung. Dieser Umstand ist insbesondere durch das unten erläuterte Beispiel 4 belegt.

Die Direktsynthese von Dimethylether wird mit fortschreitender Katalysatoralterung der Kupferkomponente des ersten Katalysators zunehmend kinetisch kontrolliert. Der Anteil an dem ersten Katalysator ist maßgeblich an der Gesamtkonversion der Direktsynthese beteiligt. Ein Verlust an Kupferaktivität hat den größten Einfluss auf die Effizienz der Direktsynthese. Höhere Anteile an dem ersten Katalysator gegen Ende des Bettes ermöglichen daher generell eine bessere Ausnutzung des Reaktors gegen das Rohrende und der dortigen durch niedrige Temperaturen und Wasserstoff- und Kohlenmonoxid-Partialdrücke beherrschte schlechtere Synthesegas Umsetzung. Dies ist insbesondere durch einen Vergleich der unten erläuterte Beispiele 2 und 3 belegt.

Es ist nicht untypisch für die im Rahmen der Erfindung eingesetzten ersten Katalysatoren, dass sie innerhalb kurzer Zeit 20 bis 30% ihrer Aktivität verlieren und im Laufe ihrer eingesetzten Zeit nur noch 25% bis 40% ihrer anfänglichen Aktivität aufweisen. Somit sind die Folgebetten vorteilhafterweise mit ca. 1,5-4-fachem Anteil dieses ersten Katalysators bezogen auf das erste Bett ausgestattet. Trotz der Alterung kann durch das strukturierte Bett für einen technischen Prozess eine höhere Konversion über einen nun längeren Zeitraum erreicht werden, da die hinteren Betten die Konversionsverlust des vorderen Bettes ausgleichen bzw. abmildern. Dies ermöglicht eine verlängerte Ausnutzung des Bettes.

Die Methanolreaktion wird gegen Ende des Reaktors der Dimethyletherreaktion gegenüber begünstigt. Etwaige höhere Selektivitäten zu Methanol stellen für die erfinderische Lösung kein Problem dar, da Methanol durch die Dimethylether-Gleichgewichtsreaktion ohnehin in der Regel in Dimethylethersyntheseverfahren recycliert wird. Der hohe Dehydratisierungskatalysatoranteil am Bettanfang stellt somit eine Konversionsmöglichkeit für recycliertes Methanol dar und verhindert gleichzeitig ein Inkonkurrenztreten der recyclierten Mengen an Methanol mit der Gleichgewichtsreaktion der Methanolsynthese. Dies ist insbesondere durch das unten erläuterte Beispiel 5 belegt.

Die Temperaturkontrolle kann sowohl durch weniger Methanolkatalysator in der Mischung, also Anpassung des Mischungsverhältnisses, als auch durch die Verwendung eines weniger aktiven Dehydratisierungskatalysators bei gleichbleibenden Methanolgehalt in der Mischung erfolgen.

Um besonders hohe Methanolbildungsraten bzw. Raum-Zeit-Ausbeuten zu realisieren, kann neben der Erhöhung des Gehalts an erstem Katalysator insbesondere ein zweiter Katalysator mit besonders hoher Aktivität eingesetzt werden, um recycliertes Methanol weitestgehend zu minimieren. Weiterreaktion des DME zu Olefinen ist in diesem Fall zu vermeiden. Durch die Anwendung des strukturierten Bettes können diese Nebenreaktion durch die Kontrolle der Reaktionstemperatur vermieden werden. Derart erhöhte Nebenproduktbildung würde zur Verkokung des Dehydratisierungskatalysators führen und zu einer rapiden Abnahme dessen Aktivität, wie sie beispielsweise aus Methanol-to-Olefins-Prozessen bekannt ist.

Die erfindungsgemäß vorgeschlagene Anlage zur Synthese von Dimethylether, die einen gekühlten Rohrbündelreaktor mit Reaktionsrohren aufweist, die mit einem ersten Katalysator und einem zweiten Katalysator ausgestattet sind, ist dafür eingerichtet, ein Kohlendioxid und/oder Kohlenmonoxid sowie Wasserstoff enthaltendes Einsatzgasgemisch durch die Reaktionsrohre des Rohrbündelreaktors zu führen, das in dem Einsatzgasgemisch enthaltene Kohlendioxid und/oder das in dem Einsatzgasgemisch enthaltene Kohlenmonoxid zumindest zum Teil an dem ersten Katalysator mit dem in dem Einsatzgasgemisch enthaltenen Wasserstoff zu Methanol umzusetzen, und bei dem in demselben

Rohrbündelreaktor das Methanol zumindest zum Teil an dem zweiten Katalysator zu dem Dimethylether umzusetzen.

Erfindungsgemäß sind die Reaktionsrohre mit dem ersten und dem zweiten Katalysator in Form einer strukturierten Schüttung ausgestattet, wobei die strukturierte Schüttung zwei bis vier Bereiche aufweist, in denen der erste und zweite Katalysator in unterschiedlichen physikalischen Mischungen bereitgestellt sind, wobei die Bereiche derart angeordnet sind, dass die Konzentration des ersten Katalysators in einer Strömungsrichtung durch die Reaktionsrohre zunimmt.

Eine erfindungsgemäße Anlage zur Herstellung von Dimethylether umfasst Mittel, die die Anlage zur Durchführung eines wie vorstehend beschriebenen Verfahrens ertüchtigen. Die erfindungsgemäße Anlage bzw. deren vorteilhafte Weiterbildungen und Ausgestaltungen profitieren dementsprechend von den Vorteilen des jeweils entsprechenden Verfahrens in analoger Weise und umgekehrt.

Im Folgenden werden weitere Merkmale und Vorteile der Erfindung sowie Ausführungsformen anhand von Ausführungsbeispielen unter Bezugnahme auf die beiliegenden Zeichnungen sowie anhand von Ausführungsbeispielen näher erläutert.

## Kurze Beschreibung der Figuren

Figur 1 zeigt eine Anlage gemäß einer Ausgestaltung der Erfindung in vereinfachter schematischer Darstellung.

Figur 2 zeigt eine Kohlenwasserstoffselektivität in Abhängigkeit der durchschnittlichen Reaktortemperatur gemäß einem Ausführungsbeispiel.

In den Figuren sind identische bzw. einander funktional entsprechende Elemente mit identischen Bezugszeichen referenziert. Erläuterungen bezüglich Verfahrensschritten gelten in entsprechender Weise für Vorrichtungskomponenten und umgekehrt.

## Ausführungsform(en) der Erfindung

In Figur 1 ist eine Anlage von Dimethylether stark vereinfacht veranschaulicht und insgesamt mit 100 bezeichnet. Die Anlage umfasst einen mit einem Kühlmittel C gekühlten Rohrbündelreaktor 10, der eine Anzahl von Reaktionsrohren 1 aufweist. Das Kühlmittel C wird im Gegenstrom zu einem Einsatzgasgemisch E, das durch die Reaktionsrohre 1 geführt wird, durch einen Mantelraum des Rohrbündelreaktors 10 geführt, optional auch in Form mehrerer Kühlmittelkreisläufe. Ein Produktgemisch P wird dem Rohrbündelreaktor 10 entnommen.

Die Reaktionsrohre 1 sind mit einem ersten und einem zweiten Katalysator in Form einer strukturierten Schüttung ausgestattet, wobei die strukturierte Schüttung im hier dargestellten Beispiel drei Bereiche 11, 12, 13 aufweist, in denen der erste und zweite Katalysator in unterschiedlichen physikalischen Mischungen bereitgestellt sind, wobei die Bereiche 11, 12, 13 derart angeordnet sind, dass die Konzentration des ersten Katalysators in einer Strömungsrichtung durch die Reaktionsrohre 1 zunimmt.

## Beispiele 1 bis 3

Im Rahmen der Beispiele 1 bis 3 wurde eine Direktsynthese mittels eines Katalysatorbettes gemäß Stand der Technik gemäß WO 2019/122078 A1 vorgenommen. Demgegenüber wurden Direktsynthesen mittels erfindungsgemäß strukturierter Betten unter ähnlichen Bedingungen durchgeführt. Die strukturierten Betten bestanden aus zwei unterschiedlich Bereichen mit Mischungen aus erstem und zweitem Katalysator. Die vordere Zone lag dabei im Bereich von 0 bis 30% der Rohrlänge der Reaktionsrohre. Die ersten 0-30% unterscheiden sich immer von den restlichen 25-100%. Die Parameter sind in Tabelle 3 zusammengefasst, wobei in der zweiten Tabellenspalte der Gesamtgehalt an dem ersten Katalysator in Gewichtsprozent, der dritten Tabellenspalte die stündliche Gas-Raumgeschwindigkeit (GHSV), der fünften Tabellenspalte die Umsetzung von Kohlenmonoxid in Prozent, der sechsten Tabellenspalte die Raumzeitausbeute (RZA) von Methanol und Dimethylether als Dimethyletheräquivalente in Gramm Dimethylether pro Kilogramm Katalysator pro Stunde und in der siebten Tabellenspalte die Selektivität zu Kohlenwasserstoffen angegeben ist.

Durch die bei der Bestimmung der Raumzeitausbeute verwendeten Dimethyletheräquivalente wird gewissermaßen auch Methanol als Dimethylether betrachtet und zur Ausbeute hinzugerechnet. Hierzu kann folgende Gleichung verwendet werden:

$$\frac{\left(\dot{n}_{DME}\left[\frac{mol}{h}\right]+\frac{1}{2}\times\dot{n}_{MeOH}\left[\frac{mol}{h}\right]\right)\times M_{DME}\left[\frac{g}{mol}\right]}{m_{Kataqlysator}\quad[kg]}$$

**Tabelle 3**

| Bsp. | Erster Kat. [Gew.-%] | GHSV [1/h] | Druck [bara] | Konv.CO [%] | RZA DME [g/(kg$_\times$h)] | Sel. KW [%] |
|---|---|---|---|---|---|---|
| 1 | 48% | 2000 | 65 | 77,1% | 320 | 2,4% |
| 2 | 46% | 3000 | 65 | 77.% | 480 | 0,7% |
| 3 | 63% | 3000 | 55 | 79,1% | 480 | 1,5% |

In Beispiel 1 wurde ein verdünntes Methanolvorbett und ein darauffolgendes gemischtes Katalysatorbett gemäß Stand der Technik eingesetzt. Beispiel 1 wurde mit einem Anteil des ersten Katalysators von 48 Gew.-%, bei einem Druck von 65 bar (abs.), einer GHSV von 2.000 $h^{-1}$, einem Verhältnis von Wasserstoff zu Kohlenmonoxid von 1,5 und einer Stöchiometriezahl von 1,2 durchgeführt. Die Konversion von Kohlenmonoxid lag bei 77,1%, die Selektivität zu Dimethylether bei 62,3 die Selektivität zu Methanol bei 6,8%, die Selektivität zu Kohlendioxid bei 29,2%, die Selektivität zu Kohlenwasserstoffen bei 2,4% und die Raumzeitausbeute von Methanol und Dimethylether als Dimethyletheräquivalente in Gramm Dimethylether pro Kilogramm Katalysator pro Stunde bei 320 g/(kg$_\times$h).

In Beispiel 2 wurde ein strukturiertes Bett bestehend aus zwei Bereichen mit ansteigendem Gehalt des ersten Katalysators eingesetzt. Beispiel 2 wurde mit einem Gehalt des ersten Katalysators von 46 Gew.-%, bei einem Druck von 65 bar(abs.), einer GHSV von 3.000 $h^{-1}$, einem Verhältnis von Wasserstoff zu Kohlenmonoxid von 1,5 und einer Stöchiometriezahl von 1,2 durchgeführt. Die Konversion von Kohlenmonoxid lag bei 77.1%, die Selektivität zu Dimethylether bei 62,2 die Selektivität zu Methanol bei 6,6%, die Selektivität zu Kohlendioxid bei 29,3%, die Selektivität zu Kohlenwasserstoffen bei 0,7% und die Raumzeitausbeute von Methanol und Dimethylether gemäß obiger Definition bei 480 g/(kg$_\times$h).

In Beispiel 3 wurden ebenfalls ein strukturiertes Bett bestehend aus zwei Bereichen mit ansteigendem Gehalt des ersten Katalysators eingesetzt. Beispiel 3 wurde mit einem Gehalt des ersten Katalysators von 68 Gew.-%, bei einem Druck von 55 bar(abs.), einer GHSV von 3.000 $h^{-1}$, einem Verhältnis von Wasserstoff zu Kohlenmonoxid von 1,5 und einer Stöchiometriezahl von 1,2 durchgeführt. Die Konversion von Kohlenmonoxid lag bei 79,1%, die Selektivität zu Dimethylether bei 60,7 die Selektivität zu Methanol bei 9,7%, die Selektivität zu Kohlendioxid bei 28,9%, die Selektivität zu Kohlenwasserstoffen bei 1,5% und die Raumzeitausbeute von Methanol und Dimethylether gemäß obiger Definition bei 480 g/(kg$_\times$h)

Der Vergleich zwischen Beispiel 1 und Beispiel 3 zeigt die erheblich bessere Raumzeitausbeute durch Verwendung eines strukturierten Bettes. Der Vergleich zwischen Beispiel 2 und Beispiel 3 zeigt, dass der Kupfergehalt maßgeblich für die Effizienz des Systems ist.

Die Tabelle 3 zeigt die Temperaturprofile zu den Beispielen 1 bis 3 und veranschaulicht, dass die strukturierten Betten gegenüber dem Stand der Technik sowohl die Temperaturkontrolle ermöglichen als auch die Raumzeitausbeute erhöhen können. Sie veranschaulicht auch das Potential, eine dritte Zone im Bereich 50 bis 100% der Rohrlänge unterzubringen, nach der Temperaturspitze bei ca. 40% des Rohres.

**Tabelle 3**

| relative Länge | [-] | 0,0 | 0,1 | 0,2 | 0,3 | 0,4 | 0,6 | 0,7 | 0,9 | 1,0 |
|---|---|---|---|---|---|---|---|---|---|---|
| T-Profil Beispiel 1 | °C | 254 | 277 | 271 | 265 | 277 | 272 | 272 | 268 | 263 |
| T-Profil Beispiel 2 | °C | 235 | 244 | 262 | 258 | 266 | 263 | 260 | 255 | 252 |
| T-Profil Beispiel 3 | °C | 236 | 247 | 274 | 262 | 273 | 266 | 258 | 255 | 250 |

Beispiel 4

In Figur 2 ist die Kohlenwasserstoffselektivität in Prozent auf der Ordinate in Abhängigkeit von der durchschnittlichen Reaktortemperatur in °C auf der Abszisse unter verschiedenen Reaktionsbedingungen, d.h. einem Verhältnis von Wasserstoff zu Kohlenmonoxid von 1,5 bis 2, einer GHSV von 2.000 bis 4.000 $h^{-1}$, einer Stöchiometriezahl von 1 bis 3 und einem Druck von 50 bis 65 bar(abs.) für unterschiedlichen Bettstrukturen und resultierende Gehalte des ersten

Katalysators mit unterschiedlichen Symbolen veranschaulicht. Dabei sind die Ergebnisse eines Vergleichs zwischen zwei strukturierten Betten (Dreiecke geben eine höhere, Quadrate eine geringere Beladung mit dem ersten Katalysator an) gegen ein Bett bestehend aus einem Methanolvorbett und darauffolgendem gemischten Katalysatorbett gemäß Stand der Technik (Kreise) verglichen.

Die Betten waren in zwei unterschiedliche katalytische Zonen eingeteilt. Die vordere Zone bestand aus ca. 30% der aktiven Rohrlänge des verwendeten gekühlten Rohrreaktors. Es zeigte sich, dass für alle Betten die Selektivität zu Nebenprodukten mit steigender Temperatur zunimmt. Zudem zeigte sich, dass die Nebenproduktbildung der strukturierten Betten mit steigendem Gehalt des ersten Katalysators zunimmt.

Darüber hinaus zeigte sich, dass die Anordnung gemäß Stand der Technik die höchste Nebenproduktbildung aufweist, obwohl ihr Gehalt an dem ersten Katalysator ähnlich wie bei dem strukturierten Bett mit dem niedrigsten Gehalt an dem ersten Katalysator ist und obwohl ihr Gesamtkupfergehalt 30% rel. niedriger ist, als jener des strukturierten Bettes mit dem höchsten Gehalt des ersten Katalysators ist.

Dies bedeutet, dass mit geeigneter Strukturierung höhere Gehalte des ersten Katalysators n ein Bett eingebracht werden können, um die Ausbeute zu erhöhen, ohne die Nebenproduktbildung zu erhöhen, oder sogar bei gleicher Ausbeute die Nebenproduktbildung zu unterdrücken.

Beispiel 5

Beispiel 5 zeigt die Möglichkeit einer Methanoldosierung am Eintritt des Katalysatorbetts ohne die Performance dieses Bettes gravierend zu stören. Das Katalysatorbett bestand aus einer 1:1 Mischung aus erstem und zweitem Katalysator. Die Ergebnisse sind in Tabelle 4 dargestellt, wobei mit x jeweils die Stoffmengenanteile dargestellt sind.

Unter identischen Bedingungen wurden zwei Referenzversuche (Tabellenspalten 1 und 3, "Standard") bei zwei unterschiedlichen Zeiten (Time on Stream, TOS), nämlich bei 193 h und 599 h) durchgeführt. Zwischen diesen Referenzen (bei 269 h) wurde ein Versuch mit Methanol im Feedgas ("Methanol-Dosierung 1") durchgeführt. Der Vergleich dieser Versuche zeigt, dass die Kohlenmonoxidkonversion nahezu unbeeinflusst ist. Dies bedeutet, dass ein Methanolrecycle hat einen geringen Einfluss unter Verwendung eines Mischkatalysatorsystems hat.

**Tabelle 3**

| Experiment | [-] | Standard | Methanol-Dosierung 1 | Standard |
|---|---|---|---|---|
| TOS | [h] | 193 | 269 | 599 |
| Druck | bar(abs.) | 60 | 60 | 60 |
| GHSV | GHSV | 3000 | 3000 | 3000 |
| x H2 Eintritt | mol/mol | 51,0% | 51,4% | 51,3% |
| x CO Eintritt | mol/mol | 25,2% | 25,5% | 24,3% |
| x CO2 Eintritt | mol/mol | 4,0% | 4,0% | 4,0% |
| x N2 Eintritt | mol/mol | 19,8% | 17,0% | 20,0% |
| x O2 Eintritt | mol/mol | 0,0% | 0,0% | 0,0% |
| x DME Eintritt | mol/mol | 0,0% | 0,0% | 0,0% |
| x MeOH Eintritt | mol/mol | 0,0% | 2,2% | 0,0% |
| Umsetzung CO | mol/mol | 71% | 70% | 70% |
| Selektivität $CO_2$ | [-] | 25% | 27% | 23% |
| Selektivität DME | [-] | 55% | 64% | 51% |
| Selektivität MeOH | [-] | 18% | 7% | 24% |
| Selektivität andere | [-] | 0,7% | 0,7% | 0,9% |
| Ausbeute DME | [-] | 39% | 45% | 35% |

**Patentansprüche**

**1.** Verfahren zur Synthese von Dimethylether, bei dem ein Kohlendioxid und/oder Kohlenmonoxid sowie Wasserstoff

enthaltendes Einsatzgasgemisch (E) durch mit einem ersten Katalysator und einem zweiten Katalysator ausgestattete Reaktionsrohre (1) eines gekühlten Rohrbündelreaktors (10) geführt wird, bei dem das in dem Einsatzgasgemisch (E) enthaltene Kohlendioxid und/oder das in dem Einsatzgasgemisch enthaltene Kohlenmonoxid zumindest zum Teil an dem ersten Katalysator mit dem in dem Einsatzgasgemisch enthaltenen Wasserstoff zu Methanol umgesetzt wird, und bei dem in demselben Rohrbündelreaktor (10) das Methanol zumindest zum Teil an dem zweiten Katalysator zu dem Dimethylether umgesetzt wird, **dadurch gekennzeichnet, dass** die Reaktionsrohre (1) mit dem ersten und dem zweiten Katalysator in Form einer strukturierten Schüttung ausgestattet sind, wobei die strukturierte Schüttung zwei bis vier Bereiche (11, 12, 13) aufweist, in denen der erste und zweite Katalysator in unterschiedlichen physikalischen Mischungen bereitgestellt sind, wobei die Bereiche (11, 12, 13) derart angeordnet sind, dass die Konzentration des ersten Katalysators in einer Strömungsrichtung durch die Reaktionsrohre (1) zunimmt.

**2.** Verfahren nach Anspruch 1, bei dem die strukturierte Schüttung als eine Aufeinanderfolge von katalytischen Bereichen mit jeweils unterschiedlichen Anteilen an dem ersten und dem zweiten Katalysator ausgebildet ist, wobei die Anteile an dem ersten und dem zweiten Katalysator innerhalb eines jeden Bereichs im Wesentlichen gleich sind.

**3.** Verfahren nach Anspruch 1 oder 2, bei dem der erste Katalysator ein kupferbasierter Katalysator ist und/oder bei dem der zweite Katalysator ein saurer Katalysator, insbesondere auf Basis eines oder mehrerer Zeolithe, von γ-Dialuminiumtrioxid und/oder von Alumosilikat, ist.

**4.** Verfahren nach einem der vorstehenden Ansprüche, bei dem der Reaktor in einem Druckbereich von 30 bis 80 bar und/oder in einem Temperaturbereich von 200 bis 290°C und/oder mit einer Gas-Raumgeschwindigkeit von 1.500 bis 4.000 $h^{-1}$ betrieben wird.

**5.** Verfahren nach einem der vorstehenden Ansprüche, bei dem die Anzahl der Bereiche (11, 12, 13), die die Schüttung aufweist, zwei ist, wobei ein erster der Bereiche (11, 12, 13) ein stromaufwärtiger Bereich von 0 bis 30% der Länge der Reaktionsrohre (1) ist und ein zweiter der Bereiche (11, 12, 13) ein stromabwärtiger Bereich von 25 bis 100% der Länge der Reaktionsrohre (1) ist.

**6.** Verfahren nach Anspruch 5, bei dem in dem ersten der Bereiche (11, 12, 13) ein Anteil des ersten Katalysators 1 und ein Anteil des zweiten Katalysators X beträgt und bei dem in dem zweiten der Bereiche (11, 12, 13) ein Anteil des ersten Katalysators 1,5 bis 4 und ein Anteil des zweiten Katalysators X beträgt.

**7.** Verfahren nach einem der Ansprüche 1 bis 4, bei dem die Anzahl der Bereiche (11, 12, 13), die die Schüttung aufweist, drei ist, wobei ein erster der Bereiche (11, 12, 13) ein stromaufwärtiger Bereich von 0 bis 30% der Länge der Reaktionsrohre (1) ist, ein zweiter der Bereiche (11, 12, 13) ein zentraler Bereich von 20 bis 70% der Länge der Reaktionsrohre (1) ist, und ein dritter der Bereiche (11, 12, 13) ein stromabwärtiger Bereich von 50 bis 100% der Länge der Reaktionsrohre (1) beträgt.

**8.** Verfahren nach Anspruch 7, bei dem in dem ersten der Bereiche (11, 12, 13) ein Anteil des ersten Katalysators 1 und ein Anteil des zweiten Katalysators X beträgt, bei dem in dem zweiten der Bereiche (11, 12, 13) ein Anteil des ersten Katalysators 1,5 bis 2 und ein Anteil des zweiten Katalysators X beträgt, und bei dem in dem dritten der Bereiche (11, 12, 13) ein Anteil des ersten Katalysators 3 bis 4 und ein Anteil des zweiten Katalysators X beträgt.

**9.** Verfahren nach einem der Ansprüche 1 bis 4, bei dem die Anzahl der Bereiche (11, 12, 13), die die Schüttung aufweist, vier ist, wobei ein erster der Bereiche (11, 12, 13) ein stromaufwärtiger Bereich von 0 bis 25% der Länge der Reaktionsrohre (1) ist, ein zweiter der Bereiche (11, 12, 13) ein stromaufwärtiger zentraler Bereich von 20 bis 50% der Länge der Reaktionsrohre (1) ist, ein dritter der Bereiche (11, 12, 13) ein stromabwärtiger zentraler Bereich von 50 bis 75% der Länge der Reaktionsrohre (1) ist, und ein vierter der Bereiche (11, 12, 13) ein stromabwärtiger Bereich von 75 bis 100% der Länge der Reaktionsrohre (1) ist.

**10.** Verfahren nach Anspruch 9, bei dem in dem ersten der Bereiche (11, 12, 13) ein Anteil des ersten Katalysators 1 und ein Anteil des zweiten Katalysators X beträgt, bei dem in dem zweiten der Bereiche (11, 12, 13) ein Anteil des ersten Katalysators 1,5 bis 2 und ein Anteil des zweiten Katalysators X beträgt, bei dem in dem dritten der Bereiche (11, 12, 13) ein Anteil des ersten Katalysators 2,5 bis 3 und ein Anteil des zweiten Katalysators X beträgt, und bei dem in dem vierten der Bereiche (11, 12, 13) ein Anteil des ersten Katalysators 4 und ein Anteil des zweiten Katalysators X beträgt.

**11.** Verfahren nach einem der Ansprüche 6, 8 oder 10, bei dem X 0,3 bis 1 beträgt.

**12.** Anlage (100) zur Synthese von Dimethylether, die einen gekühlten Rohrbündelreaktor (10) mit Reaktionsrohren (1) aufweist, die mit einem ersten Katalysator und einem zweiten Katalysator ausgestattet sind, und die dafür eingerichtet ist, ein Kohlendioxid und/oder Kohlenmonoxid sowie Wasserstoff enthaltendes Einsatzgasgemisch (E) durch die Reaktionsrohre (1) des Rohrbündelreaktors (10) zu führen, das in dem Einsatzgasgemisch (E) enthaltene Kohlendioxid und/oder das in dem Einsatzgasgemisch enthaltene Kohlenmonoxid zumindest zum Teil an dem ersten Katalysator mit dem in dem Einsatzgasgemisch enthaltenen Wasserstoff zu Methanol umzusetzen, und bei dem in demselben Rohrbündelreaktor (10) das Methanol zumindest zum Teil an dem zweiten Katalysator zu dem Dimethylether umzusetzen, **dadurch gekennzeichnet, dass** die Reaktionsrohre (1) mit dem ersten und dem zweiten Katalysator in Form einer strukturierten Schüttung ausgestattet sind, wobei die strukturierte Schüttung zwei bis vier Bereiche (11, 12, 13) aufweist, in denen der erste und zweite Katalysator in unterschiedlichen physikalischen Mischungen bereitgestellt sind, wobei die Bereiche (11, 12, 13) derart angeordnet sind, dass die Konzentration des ersten Katalysators in einer Strömungsrichtung durch die Reaktionsrohre (1) zunimmt.

**13.** Anlage (100) nach Anspruch 12, die zur Durchführung eines Verfahrens nach einem der vorstehenden Ansprüche eingerichtet ist.

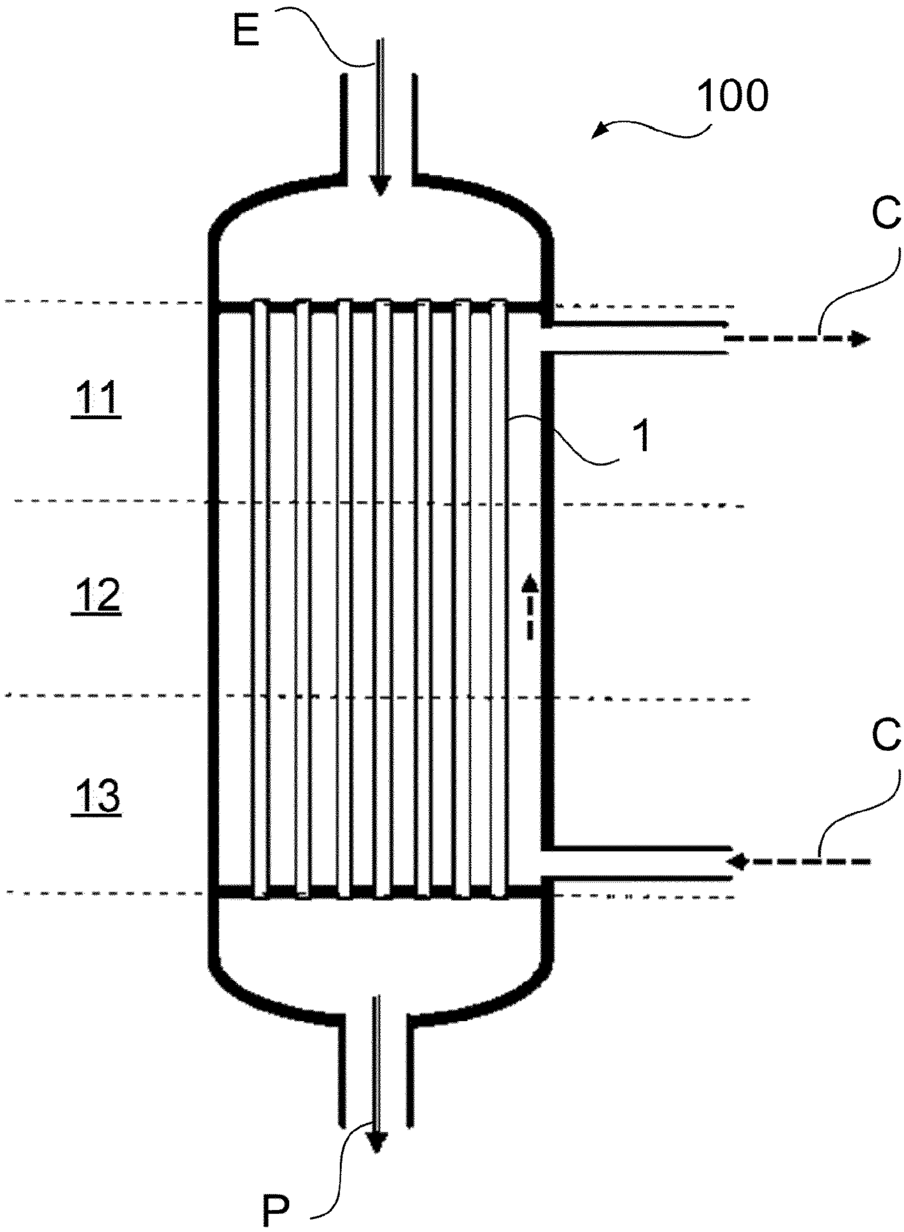


Fig. 1

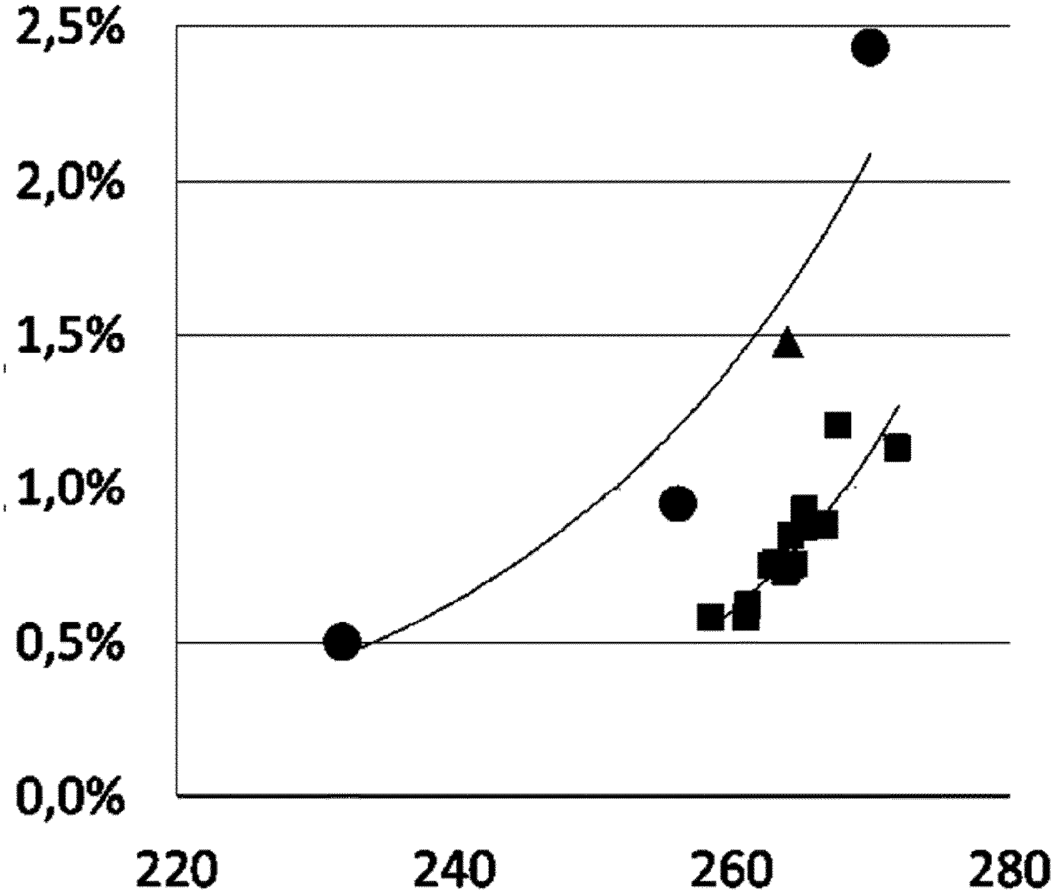


# Fig. 2

Europäisches Patentamt
European Patent Office
Office européen des brevets

# EUROPÄISCHER RECHERCHENBERICHT

Nummer der Anmeldung
EP 21 02 0619

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DELGADO OTALVARO NIRVANA ET AL: "Optimization of the direct synthesis of dimethyl ether from CO 2 rich synthesis gas: closing the loop between experimental investigations and model-based reactor design", REACTION CHEMISTRY & ENGINEERING, Bd. 5, Nr. 5, 1. April 2020 (2020-04-01), Seiten 949-960, XP055921457, DOI: 10.1039/D0RE00041H * Seite 956 - Seite 957 * * Abbildungen 5,7 * -& Nirvana Delgado ET AL: "Optimization of the direct synthesis of dimethyl ether from CO 2 rich synthesis gas: Closing the loop between experimental investigations and model-based reactor design Electronic Supplementary Information", , 1. April 2020 (2020-04-01), XP055921496, Gefunden im Internet: URL:https://www.rsc.org/suppdata/d0/re/d0re00041h/d0re00041h1.pdf [gefunden am 2022-05-16] * Abbildung 1 * | 1-13 | INV. C07C41/01 C07C43/03 B01J8/04 |
| A,D | WO 2019/122078 A1 (BASF SE [DE]) 27. Juni 2019 (2019-06-27) * Ansprüche; Beispiele * * Seite 10, Zeilen 40-41 * * Seite 11, Zeilen 10-12 * | 1 | RECHERCHIERTE SACHGEBIETE (IPC) C07C B01J |
| A,D | GERHART EIGENBERGER: "Fixed-Bed Reactors", 1. Januar 2005 (2005-01-01), ULLMANN'S ENCYCLOPEDIA OF INDUSTRIAL CHEMISTRY, WILEY-VCH VERLAG GMBH & CO. KGAA, WEINHEIM, PAGE(S) 1 - 41, XP002505406, ISBN: 978-3-527-31097-5 * das ganze Dokument * | 1 | |

Der vorliegende Recherchenbericht wurde für alle Patentansprüche erstellt

1

| Recherchenort | Abschlußdatum der Recherche | Prüfer |
|---|---|---|
| Den Haag | 20. Mai 2022 | Kardinal, Siegmar |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

# ANHANG ZUM EUROPÄISCHEN RECHERCHENBERICHT ÜBER DIE EUROPÄISCHE PATENTANMELDUNG NR.

EP 21 02 0619

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

20-05-2022

| Im Recherchenbericht angeführtes Patentdokument | Datum der Veröffentlichung | Mitglied(er) der Patentfamilie | Datum der Veröffentlichung |
|---|---|---|---|
| WO 2019122078 A1 | 27-06-2019 | CA 3086707 A1 | 27-06-2019 |
| | | CN 111491732 A | 04-08-2020 |
| | | DK 3727688 T3 | 25-04-2022 |
| | | EP 3727688 A1 | 28-10-2020 |
| | | JP 2021508286 A | 04-03-2021 |
| | | RU 2020123775 A | 20-01-2022 |
| | | US 2020316570 A1 | 08-10-2020 |
| | | WO 2019122078 A1 | 27-06-2019 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4536485 A
- US 5189203 A
- WO 2019122078 A1


### In der Beschreibung aufgeführte Nicht-Patentliteratur


- *DME Handbook,* 2007, ISBN 978-4-9903839-0-9
- **I. KIENDL et al.** *Chem. Ing. Tech.,* 2020, vol. 92 (6), 736-745
- **HILLER et al.** Gas Production. *Ullmann's Encyclopedia of Industrial Chemistry,* 15. Dezember 2006
- **A. PRAŠNIKAR et al.** *Ind. Eng. Chem. Res.,* 2019, vol. 58, 13021
- **Z.-J. ZUO et al.** *J. Phys. Chem. C,* 2014, vol. 118, 12890
- **J.J. SPIFEY ; A. EGBEBI.** *Chem. Soc. Rev.,* 2007, vol. 36, 1514
- **G. EIGENBERGER.** Fixed-Bed Reactors. *Ullmann's Encyclopedia of Industrial Chemistry,* 1992, vol. 4, 199-238